## Europäisches Patentamt
## European Patent Office
## Office européen des brevets

(11) Veröffentlichungsnummer: **0 110 287**
**B1**

(12) # EUROPÄISCHE PATENTSCHRIFT

(45) Veröffentlichungstag der Patentschrift:
**24.09.86**

(21) Anmeldenummer: **83111627.2**

(22) Anmeldetag: **22.11.83**

(51) Int. Cl.⁴: **D 02 G  3/36,** D 02 G  3/40,
A 61 L  15/00, A 62 D  5/00,
A 62 B  11/00, A 62 B  19/00

(54) **Garn mit spezifischen Eigenschaften.**

(30) Priorität: **24.11.82  DE 3243484**

(43) Veröffentlichungstag der Anmeldung:
**13.06.84 Patentblatt 84/24**

(45) Bekanntmachung des Hinweises auf die Patenterteilung:
**24.09.86 Patentblatt 86/39**

(84) Benannte Vertragsstaaten:
**AT BE CH DE FR GB IT LI LU NL SE**

(56) Entgegenhaltungen:
EP-A-0 037 745
DE-A-1 932 539
DE-A-2 055 909
DE-A-2 361 866
DE-A-2 720 633
DE-A-3 039 420
DE-B-2 804 154
FR-A-1 286 222
FR-A-2 028 162
FR-A-2 427 101
GB-A-689 565
US-A-2 230 903
US-A-3 922 723
US-A-4 010 308
US-A-4 159 618

(73) Patentinhaber: **von Blücher, Hubert, Freytagstrasse
45, D-4000 Düsseldorf (DE)**
Patentinhaber: **von Blücher, Hasso, Sohnstrasse 58,
D-4000 Düsseldorf (DE)**
Patentinhaber: **de Ruiter, Ernest, Dr.,
Höhenstrasse 57a, D-5090 Leverkusen 3 (DE)**

(72) Erfinder: **von Blücher, Hubert, Freytagstrasse 45,
D-4000 Düsseldorf (DE)**
Erfinder: **von Blücher, Hasso, Sohnstrasse 58,
D-4000 Düsseldorf (DE)**
Erfinder: **de Ruiter, Ernest, Dr., Höhenstrasse 57a,
D-5090 Leverkusen 3 (DE)**

(74) Vertreter: **Eggert, Hans- Gunther, Dr.,
Räderscheidtstrasse 1, D-5000 Köln 41 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft Garne, in denen Wirkstoffe mit spezifischen Eigenschaften in einer das Garn ummantelnden Schicht vorliegen. Die Erfindung betrifft ferner die Verwendung solcher Garne zur Herstellung von textilen Flächengebilden, flexiblen Flächenfiltern oder Schutzanzügen bzw. diese unter Verwendung des Garns hergestellten Gegenstände.

Textile Flächenfilter mit Adsorptionseigenschaften werden in den verschiedensten technischen Gebieten eingesetzt. Sie finden beispielsweise in Abzugshauben für Küchen, in Klima-Anlagen für Gebäude oder Fahrzeuge, in Masken oder Schutzanzügen gegen Giftstoffe Verwendung.

Es gibt prinzipiell zwei verschiedene Typen von Flächenfiltern. Ein bekannter Typ besteht aus einem Gewebe oder einem Vlies, beispielsweise aus Cellulose oder PAC-Fasern, wobei das Gewebe bzw. das Vlies carbonisiert und anschliessend aktiviert worden ist. Ein derartiger Flächenfilter besitzt zwar eine hohe Aktivität, jedoch ist die Festigkeit oft nicht ausreichend, wodurch die Einsatzmöglichkeit eines derartigen Filters beschränkt ist. Man ist deshalb dazu übergegangen, diesen Typ eines Flächenfilters in einer sogenannten Sandwich-Bauweise, eingebettet zwischen zwei Vliesen, anzuordnen, aber auch dann beschränkt sich der Einsatz dieser Flächenfilter immer noch hauptsächlich auf stationäre Anlagen.

Beispielsweise zur Herstellung von Schutzanzügen werden jedoch Flächenfilter gefordert, die eine ausreichende Biegsamkeit bei gleichzeitig hoher Festigkeit verlangen. Für diese Einsatzzwecke sind Flächenfilter bekannt, die aus einem mit Adsorbern beladenen flexiblen Tragermaterial, z.B. einem textilen Gewebe, einem Vlies oder einem Schaumstoff bestehen. Zum "Beladen" der Tragermaterialien mit Substanzen mit Schutzund/oder Adsorptionseigenschaften werden jedoch Bindemittel benötigt, um diese Substanzen mit dem Trägermaterial zu verbinden. Dabei wird durch das Bindemittel jedoch gleichzeitig ein beträchtlicher Teil des Porenvolumens des Trägermaterials verschlossen, wodurch die Aktivität derartiger Flächenfilter beschränkt und oft unzureichend ist.

Flammwidrig ausgerüstete textile Flächengebilde spielen bei der Raumausstattung von Hotels, Theatern und Kongreßzentren, bei Bussen und Flugzeugen eine große Rolle als präventiver Schutz. Abgesehen von flammwidrigen Ausrustungen, die jeweils nur auf eine Faser, z.B. Cellulosefasern oder Wolle ansprechen, verändern alle anderen Flammschutzmittel den Charakter von Textilien nachteilig. Der Griff wird hart bzw. brettig, und die Fähigkeit Luft und Wasserdampf hindurch zu lassen bzw. Feuchtigkeit auch nur zu transportieren, wird stark beeinträchtigt. Demzufolge leiden die Trageeigenschaften beträchtlich. Der Grund dafür ist insbesondere bei dem Bindemittel zu suchen, das das pigmentformige Flammschutzmittel fixiert aber auch das Gewebe verklebt.

Das gilt auch für die Fasern der US-A-4010308, die mit einem Schaumstoff überzogen sind, dessen offene Poren u.a. auch mit Flammschutzmitteln oder Aktivkohle gefüllt sein können. Eine Umspinnung oder Beflockung ist nicht vorgesehen und letzteres ist auch nicht möglich ohne zunächst den Flockkleber aufzubringen, der die Wirksubstanzen dann zwangsläufig bedecken wurde. Die Menge an Aktivkohle oder Flammschutzmittel, die nach dieser Patentschrift in den offenen Poren der Schaumstoffumhüllung untergebracht werden kann, ist zwangsläufig sehr gering.

Der Erfindung liegt die Aufgabe zugrunde, Garne mit spezifischen aktiven Eigenschaften, wie z.B. der Flammwidrigkeit oder der Adsorptionsfähigkeit zur Verfugung zu stellen, aus denen textile Flächengebilde hergestellt werden können die eine gute Flexibilität und Festigkeit mit hoher Aktivität für die jeweils gewünschten Wirkstoffe verbinden und damit ihre Verwendbarkeit für die verschiedensten Einsatzzwecke, z.B. als Flächenfilter oder die Trageeigenschaften von Schutzanzügen verbessern.

Gegenstand der Erfindung ist ein Garn mit spezifischen aktiven Eigenschaften, welches ein Trägergarn und eine das Tragergarn umgebende, aktive Wirkstoffe enthaltende Ummantelung aufweist, und das sich dadurch auszeichnet, daß die Ummantelung aus einem polymeren Bindemittel und dem aktiven Wirkstoff besteht, und daß die Ummantelung mit Fasern umsponnen oder beflockt ist.

Gegenstand der Erfindung sind ferner mit Hilfe eines solchen Garnes hergestelle textile Flächengebilde, insbesondere flexible Flächenfilter, und Schutzanzüge.

Die für die spezifischen Eingenschaften verantwortlichen Wirkstoffe, die in der das Garn ummantelnden Schicht vorliegen, können Adsorbenzien, insbesondere Aktivkohle, Flammschutzmittel, insbesondere Radikalfanger oder Dammschichtbildner, Dekontaminierungsmittel für chemische Kampfstoffe, Ionenaustauscher, Graphit- oder Metallpulver, Katalysatoren oder fixierte Enzyme sein.

Die aktiven Wirkstoffe können entweder in das polymere Bindemittel der Ummantelung eingebettet oder damit an dem Trägergarn fixiert sein.

Für das Trägergarn gelten weder hinsichtlich des Aufbaus noch seiner chemischen Natur besondere Einschränkungen. Es muß lediglich genügend fest sein und sollte eine gewisse Rauheit bzw. Strukturierung oder Texturierung haben damit die Ummantelung gut haftet. Das Trägergarn kann sowohl ein monofiles als auch ein multifiles Garn aus Mineral-, Synthese- oder Naturfasern sein. Die Verwendung von schwer entflammbaren bzw. besonders reißfesten Garnen aus Aramiden (KEVLAR und NOMEX) oder Polyimiden kann für bestimmte Verwendungszwecke nützlich sein. Auch Metallfasern oder metallisierte Garne können als Trägergarn dienen.

Die Binder oder Kleber fur die Fixierung der Wirkstoffe auf dem Garn sind insbesondere polymere Bindemittel, z.B. in Form von Polyurethan- oder Polyacrylatlatices, dem Latex von gegebenenfalls halogenierten Elastomerer, wie Natur-, Synthese- oder Siliconkautschuk, Chloropren oder Fluoropren. Zur Entfernung der flüchtigen Latexbestandteile müssen diese Überzüge in einem späteren Verfahrensgang getrocknet werden. Wann das geschieht hängt davon ab, welche Funktion der Binder oder Kleber noch zu erfüllen hat. Wenn die Wirkstoffe nur äußerlich an dem Kleber haften sollen, wird man die Trocknung je nach Kleber-Typ vor oder nach dem Aufbringen der Wirkstoffe vornehmen. Wenn aber die Wirkstoffe als Gemisch mit dem Binder aufgebracht werden, d.h. in das Bindemittel eingebettet sind und dieses dann auch noch zur Verankerung der Flockfasern dienen soll, wird die Trocknung zweckmäßig erst mit dem ummantelten und äußerlich beflockten Garn vorgenommen.

Je nach der chemischen Natur des Bindemittels und der Weiterverarbeitung kann außer dem Trocknen auch eine Wärmeeinwirkung zur Vernetzung bzw. Vulkanisierung der polymeren Bindemittel vorgesehen sein. Gut geeignet sind ferner Schmelzkleber auf Basis von Polyamiden, Polyestern oder Äthylen-Vinylacetat-Copolymeren (EVA). Auch diese werden für die Zwecke der Erfindung vorwiegend als Dispersionen eingesetzt. Der Gehalt der Bindemittel kann 5 bis 200, insbesondere 10 bis 100 Gewichtsprozent, bezogen auf den Wirkstoff ausmachen.

Je nach Verwendungszweck kann das Garn zuerst mit einem Kleber ummantelt und dann mit den Wirkstoffen beaufschlagt werden oder aber das Garn wird direkt mit einer paste aus dem Bindemittel und den Wirksubstanzen ummantelt. Für den Kleber- bzw. Pastenauftrag eignen sich insbesondere Vorrichtungen wie sie zum Auftrag des Flockklebers oder Flockbinders bei der Garnbeflockung Verwendung finden. Sie bestehen üblicherweise aus einem Trog, in dem das Garn benetzt wird, einem Walzenpaar mit Rillen, das den überschüssigen Kleber abstreift, und einer Trockenstrecke. Wenn die pulverförmigen Wirkstoffe äußerlich an dem Kleber auf der Faser haften sollen, wird das Trocknen selbstverständlich nach dem Aufbringen des Wirkstoffes vorgenommen.

Das auf die eine oder andere Weise mit den Wirkstoffen ummantelte Garn wird anschließend zwecks besserer Verarbeitung aber auch zur Erhöhung der Abriebfestigkeit zweckmäßig noch umsponnen oder beflockt.

Das Umspinnen des ummantelten Garns mit einem feintitrigen Endlosgarn geschieht in der Weise wie dies z.B. für das Umspinnen der Elastomerfäden von Miederware bekannt ist. Das Aussehen des Garns wird dann weitgehend durch das Umspinnen geprägt. Auch wird die Weiterverarbeitung zu textilen Flächengebilden, insbesondere dann wenn die Ummantelung verhältnismäßig rauh ist, durch das Umspinnen wesentlich erleichtert.

Anstelle des Umspinnens kann das mit den Wirkstoffen ummantelte Garn auch beflockt werden, wobei der Zweck, nämlich die Farbgebung, der Schutz der Ummantelung, die Verbesserung der Verarbeitbarkeit der gleiche ist wie bei dem Umspinnen. Die Beflockung kann gleichzeitig mit der Beladung der Kleberschicht mit den pulverförmigen Wirkstoffen, oder direkt nach dem Aufbringen einer Paste aus Wirkstoff und Bindemittel, also vor dem Trocknen der Ummantelung erfolgen. Im letztgenannten Fall kann ein hoher Anteil der Wirkstoffe in der Ummantelung eine zusätzliche Kleberschicht erfordern. Die Beflockung erfolgt in der bei Garnen üblichen Weise mit kurzstengeligen monofilen Textilfasern einer Länge von oft nur Bruchteilen von Millimetern und meist elektrostatisch. Als Flockenmaterial kommen grundsätzlich alle Natur- oder Chemiefaserstoffe infrage. Der Faserstaub besteht meistens aus Baumwolle, Wolle, Seide, Viskose, Kunstseide oder Polyamidfasern.

In einer bevorzugten Ausführung der Erfindung ist der Wirkstoff ein Adsorbens, insbesondere Aktivkohle. Als Adsorbens kommen bekannte adsorbierende Materialien wie zum Beispiel Kieselsäurexerogele, Metalloxide und -hydroxide, insbesondere Aluminiumoxid und -hydroxid, Molekularsiebe, Ionenaustauscher und Aktivkohlen verschiedener Provenienzen infrage. Die Aktivkohle kann aus geeigneten organischen Materialien in bekannter Weise hergestellt werden. Eine sehr brauchbare Aktivkohle erhält man beispielsweise durch die Carbonisierung von Ionenaustauschern auf Basis von Polystyrol und anschließende Aktivierung mit Wasserdampf. Die innere und äußere Oberfläche der Adsorbenzien kann mit Additiven, wie Schwermetallkatalysatoren oder flammhemmenden, antibakteriellen oder fungiziden Substanzen beladen sein.

Für die Verarbeitung und die Anwendung kann es sehr zweckmäßig sein, die Adsorbensteilchen, insbesondere die Aktivkohlepartikelchen, mit einer dünnen Schicht aus Polymeren zu umhüllen, die eine selektive Durchlässigkeit für die zu adsorbierenden Substanzen besitzt. So gelingt es beispielsweise, Aktivkohleteilchen durch Vorbehandlung mit Acrylatdispersionen, z.B. Acronal 50 D und 27 D der BASF, mit einem Häutchen aus Makromolekülen zu umgeben, das zwar für Kampfstoffe durchlässig ist, jedoch nicht für zahlreiche, die Aktivkohle blockierenden Stoffe wie z.B. Schweiß. Eine andere Möglichkeit die Adsorbenskörner zu inkapsulieren, besteht darin, daß man sie in einem Zweikomponentengebläse mit Polyamidpulver möglichst gleicher Teilchengröße etwa im Mengenverhältnis 1:1 verwirbelt und durch einen Gasflammenrohrofen gegen ein gekühltes, spiegelverchromtes Stahlblech schleudert. Das im Glasflammenrohrofen erweichte Polyamid legt sich im Luftstrom um das Adsorberteilchen und verfestigt sich dort beim Austritt aus dem Flammrohr im gekühlten Luftstrom.

Jeder Verklebung der so mit Polyamid umhüllten Adsorbenskorner untereinander wird durch Auftreffen auf das bis etwa -15°C heruntergekühlte spiegelverchromte Stahlblech entgegengewirkt. Andere bekannte Prozesse zur Mikroinkapsulation der Adsorbenskörner mit verschiedenen anorganischen oder organischen Hüllmaterialien können ebenfalls verwendet werden. Durch die Inkapsulierung läßt sich erreichen, daß das Bindemittel die Aktivkohle nicht blockiert, wenn man für die Ummantelung eine Paste aus verpulverter Aktivkohle und einem Bindemittel verwendet. Um eine hohe Wirksamkeit zu gewährleisten, ist in diesem Falle das pulverformige Adsorbens sehr fein gemahlen und hat nur eine Teilchengröße von 0,01 bis 50 μm, vorzugsweise 1 bis 10 μm. Mit Strahlenmühlen läßt sich beispielsweise Aktivkohle auf Partikelgrößen von 95 % unter 3 μm bringen. Solche Aktivkohlen können vorherbestimmbare Porengrößen bei sehr hoher Aktivfläche bis zu 1.400 m²/g besitzen. Wird zuerst ein Kleber auf das Garn aufgetragen und darin dann die Aktivkohle fixiert, so werden je nach Garnstärke Teilchen von 10 bis 100 μm bevorzugt. Feiner Staub ist zu vermeiden, weil er zum Abpudern·der Kleberschicht führt.

Wenn gemäß einer anderen Ausführung der Erfindung Katalysatoren, insbesondere Metalloxide, Ionenaustauscher, Molekularsiebe oder pigmentförmige Flammschutzmittel, den Wirkstoff der Ummantelung darstellen sollen, gilt für die Art der Aufbringung und die Teilchengröße ähnliches wie für die Adsorbenzien, insbesondere die Aktivkohle.

Hinsichtlich der als Wirkstoff verwendbaren Flammschutzmittel unterliegt die Erfindung praktisch keinen Beschränkungen. Es kann sich sowohl um verkohlungsfördernde und feuererstickende als auch sperrschichtbildende Flammschutzmittel oder um Radikalfänger handeln. Gut geeignet sind auch die sogenannten Dämmschichtbildner, die Substanzen enthalten, die sich beim Erwärmen schaumig aufblähen, ab 200 bis 300°C verkohlen, sich dabei verfestigen und ein feinporiges gut isolierendes Polster bilden. Brauchbar sind ferner die für Kunststoffe bekannten Flammschutzmittel, deren flammhemmende Wirkung noch durch sogenannte Synergisten oder bestimmte Weichmacher bzw. die insbesondere bei pigmentformigen Flammschutzmitteln notwendigen Bindemittel verstärkt werden kann. Auch selbstverlöschende Kunststoffe, insbesondere solche mit höherem Halogengehalt, sind allein oder in Kombination mit anderen bekannten Flammschutzmitteln brauchbar. Zusammenfassend sind also alle Flammschutzmittel im Rahmen der Erfindung verwendbar, die in Kombination mit dem als Träger dienenden textilen Flächengebilde und untereinander verträglich und beispielsweise in Römpps Chemie-Lexikon, 8. Aufl., (1981) unter dem Stichwort Flammschutzmittel und den hier aufgeführten Literaturstellen beschrieben sind. Die sich unter Hitzeeinwirkung schaumig aufblähenden Dämmschichtbildner entwickeln einen nicht entflammbaren isolierenden Schaum, der im Ernstfall auch unter der Umspinnung bzw. Beflockung hervorquillt und das mit einem solchen Garn hergestellte Gewebe gegen heiße Gase abdichtet.

In gleicher Weise wie Adsorbenzien oder Flammschutzmittel können auch als Wirkstoff Chemikalien in die Ummantelung eingebaut werden, die chemische Kampfstoffe zerstören, so daß man hieraus einen sich selbst dekontaminierenden Schutzanzug herstellen könnte. Der Fachmann wird Binder und Garne wählen, die mit den eingesetzten Chemikalien verträglich sind. Garne mit Ionenaustauschern ummantelt eignen sich zur Herstellung von Flächengebilden bzw. Flächenfiltern mit austauschenden Eigenschaften. In ähnlicher Weise lassen sich Flächengebilde mit katalytischen Eigenschaften herstellen, die für katalytische Prozesse, die keine hoheren Temperaturen verlangen, geeignet sind.

Besonderes Augenmerk ist Enzymen zu widmen, die oft organische Katalysatoren genannt werden, aber viel spezifischer und effizienter als herkömmliche Katalysatoren sind. Enzyme brauchen jedoch normalerweise zur Entfaltung ihrer Aktiviät Wasser. Eine Möglichkeit, diese Schwierigkeit zu umgehen, besteht darin, winzige das Enzym enthaltende Gel-Tröpfchen zu inkapsulieren und diese Mikrokapseln bei der Ummantelung einzusetzen, wobei die Inkapsulierung das Gel erhalten soll, aber die Reaktion mit dem Enzym nicht verhindern darf. Es gibt Enzyme, die chemische Kampfstoffe der Gruppe der Nervengifte sehr spezifisch attackieren; in diesem Fall wird man eine Inkapsulierung mit hydrophoben und oleophilen Eigenschaften wählen, so daß der Kampfstoff eindringen kann. Flächenfilter mit Enzymen beladen eignen sich für Schutzanzüge (C-Schutz, Nervengifte), Industriefilter, Klimaanlagen usw. Eine Variante den Enzym beinhaltenden Ummantelung besteht darin, daß das Garn vorerst durch eine schwammige Schicht umgeben wird - eine solche wurde z.B. durch ein koaguliertes polyurethan realisiert - in welche das das Enzym beinhaltende Gel eingelagert wird. Zum Schutze des Gels empfiehlt sich eine hydrophobe Deckschicht, deren Wirkungsweise bereits bei der Inkapsulierung beschrieben wurde. Schließlich hat es sich gezeigt, daß das Gel durch Zugabe nicht-flüchtiger, hygroskopischer Stoffe, die verschiedenster Natur sein können, jedoch den pH-Wert und den Reaktionsmechanismus nicht beeinträchtigen dürfen, gegenüber Luftfeuchtigkeit stabilisiert werden kann, so daß je nach Anwendung auf die das Gel schützende Schicht verzichet werden kann. Schließlich kann auch Aktivkohle als Träger für Enzyme bzw. in eine Kunststoffmatrix eingebaute Enzyme herangezogen werden. Es ist dem Fachmann bekannt, daß Enzyme gegen Mikroorganismen geschützt werden müssen.

Die Dicke der Ummantelung hängt weitgehend von der Stärke des Trägergarns ab und liegt etwa in der gleichen Größenordnung. Bevorzugt werden Garne einer Stärke von 5 bis 100 den je nach Anwendungszweck, wobei die geringere Garnstärke für Bekleidungszwecke und die hohe Garnstärke z.B. für Industriefilter gewählt wird. Die Dicke der Ummantelung beträgt insbesondere 25 bis 300 % des Garndurchmessers.

Das ummantelte, insbesondere zusätzliche umsponnene oder beflockte Garn wird dann zu einem textilen Flächengebilde verarbeitet. Die bevorzugte Art ist ein Gewebe, in dem die adsorbierenden Fäden die Kette bilden. Dabei können alle Kettfäden ummantelte Fäden nach der Erfindung sein oder es konnen auch andere Kettfäden mitverwendet werden. Die Farbe des Gewebes wird durch den Schußfaden bzw. durch das für das Umspinnen oder Beflocken eingesetzte Material bestimmt. So erhaltene Gewebe ergeben beispielsweise Flächenfilter mit bis zu 100 g Aktivkohle pro m². Die textilen und physiologischen Eigenschaften erlauben hieraus hervorragende Schutzanzüge gegen chemische Kampfstoffe herzustellen.

## Patentansprüche

1. Garn mit spezifischen aktiven Eigenschaften, welches ein Trägergarn und eine das Trägergarn umgebende, aktive Wirkstoffe enthaltende Ummantelung aufweist, dadurch gekennzeichnet, daß die Ummantelung aus einem polymeren Bindemittel und dem aktiven Wirkstoff besteht, und daß die Ummantelung mit Fasern umsponnen oder beflockt ist.

2. Garn nach Anspruch 1, dadurch gekennzeichnet, daß die Ummantelung als aktiven Wirkstoff adsorbierende Materialien enthält.

3. Garn nach Anspruch 1 oder 2, dadurch gekennzeichnet, daß die Ummantelung als aktiven Wirkstoff Aktivkohle enthält.

4. Garn nach einem der Ansprüche 1 bis 3, dadurch gekennzeichnet, daß die Ummantelung als aktiven Wirkstoff ein Flammschutzmittel, insbesondere einen Radikalfänger oder Dämmschichtbildner enthält.

5. Garn nach Anspruch 1, dadurch gekennzeichnet, daß die Ummantelung als aktiven Wirkstoff Ionenaustauscher enthält.

6. Garn nach Anspruch 1, dadurch gekennzeichnet, daß die Ummantelung als aktiven Wirkstoff ein Dekontaminierungsmittel für chemische Kampfstoffe enthält.

7. Garn nach Anspruch 1, dadurch gekennzeichnet, daß die Ummantelung als aktiven Wirkstoff einen Katalysator enthalt.

8. Garn nach Anspruch 1, dadurch gekennzeichnet, daß die Ummantelung als aktiven Wirkstoff ein fixiertes Enzym enthält.

9. Garn nach Anspruch 1, dadurch gekennzeichnet, daß die Ummantelung als aktiven Wirkstoff ein Metallpulver enthält.

10. Garn nach einem der Ansprüche 1 bis 9, dadurch gekennzeichnet, daß der aktive Wirkstoff in das polymere Bindemittel der Ummantelung eingebettet ist.

11. Garn nach einem der Ansprüche 1 bis 9, dadurch gekennzeichnet, daß der aktive Wirkstoff mit dem polymeren Bindemittel an dem Trägergarn fixiert und diese Ummantelung umsponnen ist.

12. Garn nach einem der Ansprüche 1 bis 4, dadurch gekennzeichnet, daß das Trägergarn aus Aramiden besteht.

13. Verwendung von Garn nach einem der Ansprüche 1 bis 12 zur Herstellung von textilen Flächengebilden.

14. Verwendung von Garn nach einem der Ansprüche 1 bis 12 zur Herstellung von flexiblen Flächenfiltern.

15. Verwendung von Garn nach einem der Ansprüche 1 bis 12 zur Herstellung von Schutzanzügen.

## Claims

1. Yarn with specific active properties, which has a carrier yarn and a sheathing which surrounds the carrier yarn and contains active substances, characterised in that the sheathing consists of a polymeric binding agent and of the active substance, and that the sheathing is covered with fibres by winding or flocking.

2. Yarn according to Claim 1, characterised in that the sheathing contains adsorbent materials as active substance.

3. Yarn according to Claim 1 or 2, characterised in that the sheathing contains activated carbon as active substance.

4. Yarn according to one of Claims 1 to 3, characterised in that the sheathing contains as active substance a fireproofing agent, in particular a radical inhibitor or a substance capable of forming an insulating layer.

5. Yarn according to Claim 1, characterised in that the sheathing contains ion exchangers as active substance.

6. Yarn according to Claim 1, characterised in that the sheathing contains as active substance a decontamination agent for chemical warfare substances.

7. Yarn according to Claim 1, characterised in that the sheathing contains a catalyst as active substance.

8. Yarn according to Claim 1, characterised in that the sheathing contains a fixed enzyme as active substance.

9. Yarn according to Claim 1, characterised in that the sheathing contains a metal powder as active substance.

10. Yarn according to one of Claims 1 to 9, characterised in that the active substance is embedded in the polymeric binding agent of the sheathing.

11. Yarn according to one of Claims 1 to 9, characterised in that the active substance is fixed with the polymeric binding agent on the carrier yarn and this sheathing is covered by winding.

12. Yarn according to one of Claims 1 to 4, characterised in that the carrier yarn consists of aramides.

13. Use of yarn according to one of Claims 1 to 12 for the manufacture of textile sheet products.

14. Use of yarn according to one of Claims 1 to 12 for the manufacture of flexible surface filters.

15. Use of yarn according to one of Claims 1 to 12 for the manufacture of protective clothing.

## Revendications

1. Fil présentant des propriétés actives spécifiques, comprenant un fil substrat et une enveloppe qui entoure ce fil substrat et renferme des substances actives, caractérisé par le fait que l'enveloppe est constituée par un liant polymère et par la substance active; et par le fait que l'enveloppe est guipée ou floquée avec des fibres.

2. Fil selon la revendication 1, caractérisé par le fait que l'enveloppe renferme des matériaux adsorbants en tant que substance active.

3. Fil selon la revendication 1 ou 2, caractérisé par le fait que l'enveloppe renferme du charbon actif en tant que substance active.

4. Fil selon l'une des revendications 1 à 3, caractérisé par le fait que l'enveloppe renferme un agent ignifuge en tant que substance active, en particulier un capteur de radicaux ou un formateur de couches isolantes.

5. Fil selon la revendication 1, caractérisé par le fait que l'enveloppe renferme des échangeurs d'ions en tant que substance active.

6. Fil selon la revendication 1, caractérisé par le fait que l'enveloppe renferme, en tant que substance active, un agent de décontamination pour substances chimiques de combat.

7. Fil selon la revendication 1, caractérisé par le fait que l'enveloppe renferme un catalyseur en tant que substance active.

8. Fil selon la revendication 1, caractérisé par le fait que l'enveloppe renferme une enzyme fixée, en tant que substance active.

9. Fil selon la revendication 1, caractérisé par le fait que l'enveloppe renferme une poudre métallique en tant que substance active.

10. Fil selon l'une des revendications 1 à 9, caractérisé par le fait que la substance active est noyée dans le liant polymère de l'enveloppe.

11. Fil selon l'une des revendications 1 à 9, caractérisé par le fait que la substance active est fixée sur le fil substrat avec le liant polymère, puis cette enveloppe est guipée.

12. Fil selon l'une des revendications 1 à 4, caractérisé par le fait que le fil substrat consiste en des aramides.

13. Utilisation d'un fil selon l'une des revendications 1 à 12, pour fabriquer des structures textiles planes.

14. Utilisation d'un fil selon l'une des revendications 1 à 12, pour fabriquer des filtres flexibles plans.

15. Utilisation d'un fil selon l'une des revendications 1 à 12, pour fabriquer des combinaisons protectrices.